# EUROPEAN PATENT SPECIFICATION

(11) **EP 4 210 780 B1**
(45) Date of publication and mention of the grant of the patent: **20.11.2024**
(21) Application number: 21739282.8
(22) Date of filing: 23.06.2021
(51) Int. Cl.: A61M 3/02, A61F 11/00

(54) **EAR DROP APPLICATOR**
OHRTROPFENAPPLIKATOR
APPLICATEUR DE GOUTTES AURICULAIRES

(30) Priority: 26.06.2020 GB 202009754
(43) Date of publication of application: 19.07.2023
(73) Proprietor: Gokani, Bhavesh, Watford, Hertfordshire WD25 0JL (GB)
(72) Inventor: Gokani, Bhavesh, Watford, Hertfordshire WD25 0JL (GB)
(74) Representative: Cleveland Scott York
(86) International application number: PCT/EP2021/067170
(87) International publication number: WO 2021/260016

(56) References cited:
- EP-A1- 3 590 478
- WO-A1-2017/185001
- US-A1- 2014 102 461

## Description

### Field of the Invention

This invention relates to the application of ear drops and has for its object the provision of an improved means and method for the application of ear drops.

### Relevant Background art

Relevant prior art is exemplified by US 2014/102461 A1.

### Summary of the Invention

The invention pertains to an ear drop applicator for the application of ear drops as defined in appended claim 1. Preferred embodiments are defined in the dependent claims.

According to a first aspect of the present invention there is provided an ear drop applicator for the application of ear drops, which comprises a tray for receiving a bottle of ear drops placed therein, wherein the tray comprises a spout for inserting into an ear canal and through which the ear drops can pass from the bottle into the ear canal.

The tray preferably comprises a base with an opening for facilitating gripping of the bottle by the user. The opening may comprise a cut-out in the base of the tray through which a user can press a bottle received in the tray using their thumb.

The tray may have a handle, with the spout at one end of the tray and the handle at the other end of the tray. The spout may be a conical formation and is preferably so shaped that the applicator cannot be inserted to deeply into the ear canal.

The tray may have a curved shape and may be formed as a plastic moulding.

According to another aspect of the present invention there is provided a method of applying ear drops to an ear canal using an ear drop applicator as defined above, the method comprising:
inserting a bottle of ear drops into the tray;
pushing the bottle into the spout;
engaging the bottle and the tray with the hand of a user;
inserting the spout into the ear canal; and
pressing the bottle so as to cause drops to fall from the bottle into the ear canal.

The bottle is preferably engaged by grasping the bottle with the fingers of a user's hand and with the thumb of the user's hand engaging the bottle through the opening.

According to a further aspect of the present invention there is provided a method of applying ear drops from a pipette into an ear canal of another person using an ear drop applicator as defined above, the method comprising:
inserting the spout into the other person's ear canal;
positioning the pipette above the applicator; and
releasing a drop into the spout so that it is directed into the ear canal.

### Brief Description of the Drawings

The drawings show a number of views of one form of ear drop applicator.

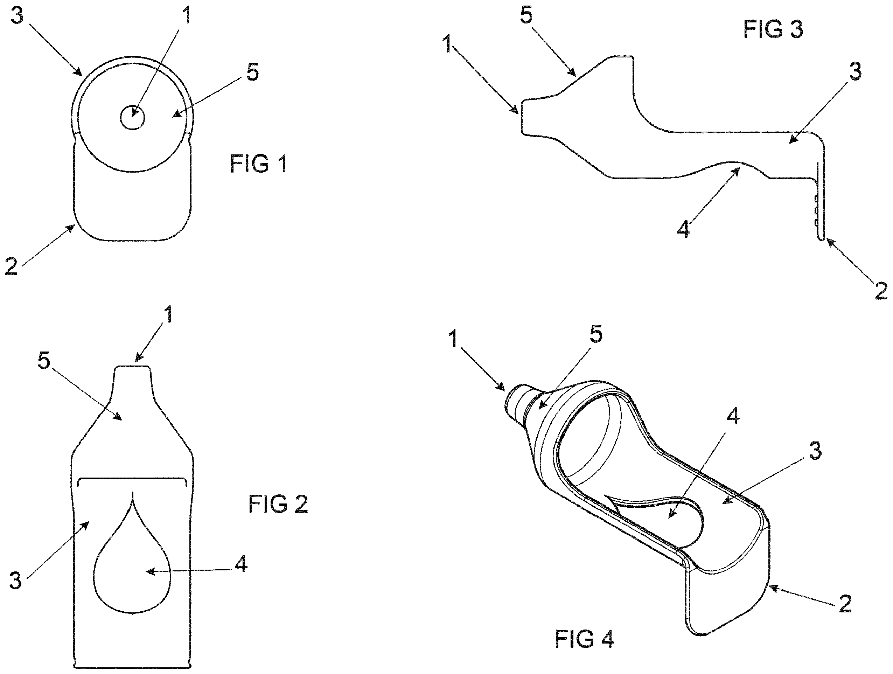

Figure 1 is a front view of the applicator,
Figure 2 is a plan view of the applicator,
Figure 3 is a side view of the applicator,
Figure 4 is perspective view of the applicator, and

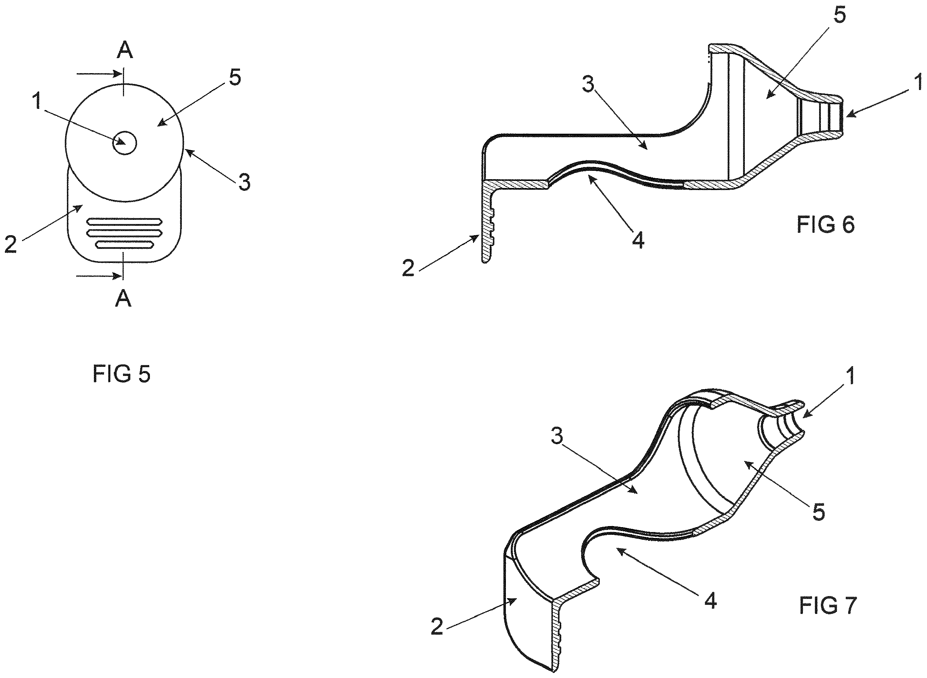

Figures 5, 6 and 7 are sectional views corresponding to Figures 1, 3 and 4.

### Description of the Preferred Embodiment

The ear drop applicator shown in the drawings is formed as a plastic moulding and comprises a tray 3 having a curvature such that it can receive and support a variety of sizes of bottles of ear drops. At one end of the tray 3, there is a downwardly extending handle 2 and, at the other end of the tray 3, there is a spout in the form of a conical formation 5 which is so shaped as to avoid the applicator being inserted too deeply into the ear canal of the person to whom the ear drops are being applied through a hole 1 at the end of the conical formation. There is a cut-out 4 in the base of the tray 3 to enable the person holding the applicator to grasp a bottle placed in the tray securely to avoid it moving about within the tray 3.

The entire shape of the applicator, particularly the shape of the conical formation 5, is such as to ensure that the applicator cannot be inserted too deeply into the ear canal.

There are two ways in which to use the applicator, i.e. self-administration and administering drops to another person. These are as follows:-

### 1) Self-Administration.

Wash the applicator to make sure that it is completely clean.

Keeping the tray 3 flat, i.e. horizontal, a bottle of ear drop solution is slid into the tray 3.

The bottle is pushed into the spout of the applicator.

The bottle is grasped by pressing with the thumb of one hand through the cut-out 4 in the base of the tray 3 and with the fingers of that hand in engagement with the bottle.

With the bottle inserted in the applicator, the conical formation 5 is pushed gently into the ear canal.

The user then tilts his or her head as far as possible or lies down and gently presses the bottle so as to cause a drop of the solution to fall from the bottle into the ear canal.

The applicator is then lifted out of the ear canal.

The user then stays still for about two minutes to ensure that the drop of solution can travel the required distance down into the ear canal.

2) Administering drops to another person, such as a patient, a child or an elderly person.

Wash the applicator to make sure that it is completely clean.

The handle 2 is gripped between the thumb and first finger with the thumb on the top of the handle 2.

Ask the other person to tilt their head to one side or to lie down with the relevant ear uppermost.

Depending on the type of bottle of ear drops that is being used, insert the bottle into the tray 3 of the applicator and gently push the top of the bottle into the conical formation 5.

If the bottle of ear drops is one that has a pipette, there will be no need to put the bottle into the tray 3.

Lift the applicator and insert the conical formation 3 in the ear canal of the other person before gently squeezing the bottle.

If using a pipette, position the pipette above the applicator and release a drop into the conical formation so that it is directed into the entrance of the ear canal.

Ask the person being treated to remain on their side or with their head tilted for about two minutes to allow gravity to take the drop through the chamber of the ear canal.

If the applicator is designed as a "single use" device, it will not require washing before use and may be produced using a suitable biodegradable material.

## Claims

1. An ear drop applicator for the application of ear drops, which comprises a tray (3), wherein the tray (3) comprises a spout for inserting into an ear canal and through which the ear drops can pass from a bottle into the ear canal; and
**characterised in that** the tray is suitable for receiving a bottle of ear drops placed therein, wherein the bottle can be pushed into the spout of the applicator, and the tray (3) comprises a base with an opening (4) for facilitating gripping of the bottle by the user.

2. An ear drop applicator as claimed in Claim 1, in which the opening comprises a cut-out (4) in the base of the tray (3) through which a user can press a bottle received in the tray (3) using their thumb.

3. An ear drop applicator as claimed in any one of the preceding claims in which the tray (3) has a handle (2).

4. An ear drop applicator as claimed in claim 3, in which the spout is at one end of the tray (3) and the handle (2) is at the other end of the tray (3).

5. An ear drop applicator as claimed in any preceding claim, in which the spout is a conical formation (5).

6. An ear drop applicator as claimed in Claim 5, in which the conical formation (5) is so shaped that the applicator cannot be inserted to deeply into the ear canal.

7. An ear drop applicator as claimed in any preceding claim, in which the tray (3) has a curved shape.

8. An ear drop applicator as claimed in any preceding claim, in which the tray (3) is formed as a plastic moulding.

## Patentansprüche

1. Ohrtropfenapplikator zur Applikation von Ohrtropfen, der eine Schale (3) umfasst,
wobei die Schale (3) eine Tülle zum Einführen in einen Gehörgang umfasst und durch welche die Ohrtropfen aus einer Flasche in den Gehörgang gelangen können; und
**dadurch gekennzeichnet, dass** die Schale zum Aufnehmen einer darin platzierten Flasche mit Ohrtropfen geeignet ist, wobei die Flasche in die Tülle des Applikators eingeschoben werden kann, und
die Schale (3) einen Boden mit einer Öffnung (4) umfasst, um Greifen der Flasche durch den Benutzer zu erleichtern.

2. Ohrtropfenapplikator nach Anspruch 1, wobei die Öffnung eine Aussparung (4) in dem Boden der Schale (3) umfasst, durch die ein Benutzer auf eine Flasche, die in der Schale (3) aufgenommen ist, unter Verwendung seines Daumens drücken kann.

3. Ohrtropfenapplikator nach einem der vorhergehenden Ansprüche, wobei die Schale (3) einen Griff (2) aufweist.

4. Ohrtropfenapplikator nach Anspruch 3, wobei die Tülle an einem Ende der Schale (3) ist und der Griff (2) an dem anderen Ende der Schale (3) ist.

5. Ohrtropfenapplikator nach einem vorhergehenden Anspruch, wobei die Tülle eine konische Formation (5) ist.

6. Ohrtropfenapplikator nach Anspruch 5, wobei die konische Form (5) so geformt ist, dass der Applikator nicht zu tief in den Gehörgang eingeführt werden kann.

7. Ohrtropfenapplikator nach einem vorhergehenden Anspruch, wobei die Schale (3) eine gekrümmte Form aufweist.

8. Ohrtropfenapplikator nach einem vorhergehenden Anspruch, wobei die Schale (3) als Kunststoffformteil gebildet ist.

## Revendications

1. Applicateur de gouttes auriculaires pour l'application de gouttes auriculaires, qui comprend un plateau (3),
dans lequel le plateau (3) comprend un bec verseur destiné à être inséré dans un conduit auditif et via lequel les gouttes auriculaires peuvent passer d'un flacon au conduit auditif ; et
**caractérisé en ce que** le plateau est adapté pour recevoir un flacon de gouttes auriculaires placé dans celui-ci, dans lequel le flacon peut être poussé dans le bec verseur de l'applicateur, et
le plateau (3) comprend une base avec une ouverture (4) destinée à faciliter la préhension du flacon par l'utilisateur.

2. Applicateur de gouttes auriculaires selon la revendication 1, dans lequel l'ouverture comprend une découpe (4) dans la base du plateau (3) à travers laquelle un utilisateur peut appuyer sur un flacon reçu dans le plateau (3) à l'aide de son pouce.

3. Applicateur de gouttes auriculaires selon l'une quelconque des revendications précédentes, dans lequel le plateau (3) comporte une poignée (2).

4. Applicateur de gouttes auriculaires selon la revendication 3, dans lequel le bec verseur se trouve à une extrémité du plateau (3) et la poignée (2) se trouve à l'autre extrémité du plateau (3).

5. Applicateur de gouttes auriculaires selon l'une quelconque des revendications précédentes, dans lequel le bec verseur est une formation conique (5).

6. Applicateur de gouttes auriculaires selon la revendication 5, dans lequel la formation conique (5) est formée de telle sorte que l'applicateur ne puisse pas être inséré trop profondément dans le conduit auditif.

7. Applicateur de gouttes auriculaires selon l'une quelconque des revendications précédentes, dans lequel le plateau (3) comporte une forme incurvée.

8. Applicateur de gouttes auriculaires selon l'une quelconque des revendications précédentes, dans lequel le plateau (3) est formé comme un moulage plastique.
